(19) Europäisches Patentamt
European Patent Office
Office européen des brevets

(11) EP 1 240 125 B2

(12) NEW EUROPEAN PATENT SPECIFICATION
After opposition procedure

(45) Date of publication and mention of the opposition decision:
**20.04.2016 Bulletin 2016/16**

(45) Mention of the grant of the patent:
**02.11.2005 Bulletin 2005/44**

(21) Application number: **00926297.3**

(22) Date of filing: **20.04.2000**

(51) Int Cl.:
***C07C 67/307*** (2006.01) ***C07C 41/18*** (2006.01)
***C07C 41/24*** (2006.01) ***C07C 41/48*** (2006.01)

(86) International application number:
**PCT/US2000/010942**

(87) International publication number:
**WO 2001/046107 (28.06.2001 Gazette 2001/26)**

(54) **FLUORINE CONTAINING VINYL ETHERS**

FLUOR ENTHALTENDE VINYLETHER

VINYL-ETHERS CONTENANT DU FLUOR

(84) Designated Contracting States:
**BE DE FR GB IT NL**

(30) Priority: **22.12.1999 US 470497**

(43) Date of publication of application:
**18.09.2002 Bulletin 2002/38**

(60) Divisional application:
**05017869.8 / 1 616 849**

(73) Proprietor: **3M Innovative Properties Company**
**Saint Paul, MN 55133-3427 (US)**

(72) Inventors:
- **WORM, Allan, T.**
**Saint Paul, MN 55133-3427 (US)**
- **MOORE, George, G., I.**
**Saint Paul, MN 55133-3427 (US)**
- **GUERRA, Miguel, A.**
**Saint Paul, MN 55133-3427 (US)**
- **SCHWERTFEGER, Werner**
**Saint Paul, MN 55133-3427 (US)**
- **HINTZER, Klaus**
**Saint Paul, MN 55133-3427 (US)**
- **QUI, Zai-Ming**
**Saint Paul, MN 55133-3427 (US)**
- **HARE, Erik, D.**
**Saint Paul, MN 55133-3427 (US)**

(74) Representative: **Kurz, Arnd et al**
**3M Deutschland GmbH**
**3M Office of Intellectual Property Counsel**
**Carl-Schurz-Strasse 1**
**41453 Neuss (DE)**

(56) References cited:

| | |
|---|---|
| EP-A- 0 290 848 | EP-A1- 0 130 052 |
| WO-A1-00/12574 | WO-A1-01/27194 |
| WO-A1-99/48939 | US-A- 3 451 908 |
| US-A- 4 906 770 | US-A- 4 981 727 |
| US-A- 5 149 842 | US-A- 5 350 497 |
| US-A- 5 589 557 | US-A- 5 696 216 |

Remarks:
The file contains technical information submitted after the application was filed and not included in this specification



Printed by Jouve, 75001 PARIS (FR)

## Description

### Field:

The present invention relates to perfluorinated vinyl ethers as valuable comonomers for fluoroelastomers with enhanced low temperature properties.

### Background:

The benefits of modifying fluoropolymers by vinyl ethers are described in various review articles. See, for example, Modem Fluoropolymers, John Scheirs, Wiley Series in Polymer Science 1997 and in other literature (e.g. Emel 'yanov et al, Zh. Org. Khim (1994) 30(8) 1266-70; Krespan, Carl G., DuPont de Nemours U. S. 4,273,728).

Partly fluorinated vinyl ethers and their copolymers are described in A. E. Feiring et al, DuPont de Nemours U.S. 5,326,917. Long chain vinyl ethers provide in fluoroelastomers excellent low temperature properties (see Uschold et al, U.S. Patent 4,513,128).

The preparation of perfluoro(alkyl vinyl ethers) by fluorination with elemental fluorine is known. See Hung et al, U.S. Patent 5,350,497. This patent discloses the fluorination of selected partially fluorinated (di)chloroethyl ethers (followed by dehalogenation to the corresponding perfluoro(alkyl vinyl ether).

Perfluorinated long chain perfluoroethers are difficult to prepare, especially those vinyl ethers without branching and more than 3 atoms in the alkyl group. For example, perfluorovinyl ethers are commonly prepared by two routes. See, for example, Modem Fluoropolymers, J. Scheirs, Wiley 1997 pp 376-378.

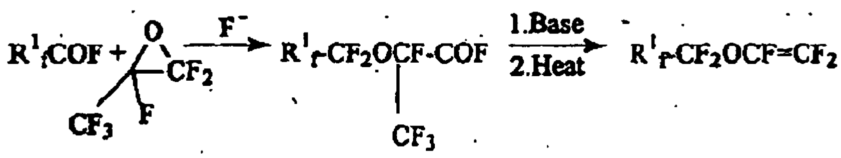


Addition of perfluorinated acid fluorides to hexafluoropropylene oxide results in an acid fluoride that may be converted to a salt and pyrolyzed to give the described perfluorovinyl ether. The oligomerization of hexafluoropropylene oxide with itself and conversion to a salt and subsequent pyrolysis gives long chain but branched ethers.

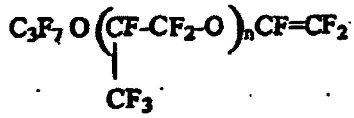

A more recent process prepares perfluorovinyl ethers by using perfluoro alkyl hypofluorites and dichlorodifluoroethylene followed by dehalogenation using, for example, Zn.

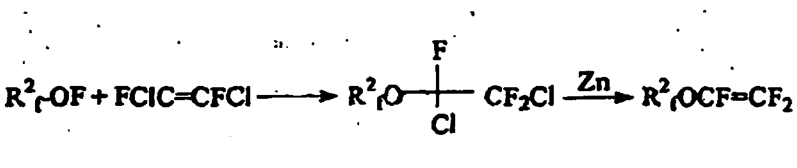


As noted, it is difficult to provide long chain perfluorinated ethers, especially vinyl ethers, when using these processes. A difficulty in the dichloro process includes the hazards of making and handling the $R^2$+OF species. Because of these hazards, this species also has a limited avaitability. An improved process for making perfluorinated vinyl ethers is needed, particularly for making linear perfluorinated vinyl ethers.

### Summary:

The present invention also relates to a fluoroelastomer as defined in the claim.

As used herein, the term perfluorinated means that all of the carbon bonded hydrogen atoms have been replaced with fluorine and any unsaturated carbon-carbon bonds have been saturated with fluorine In the following methods of making vinylether are described that include vinylethers as recited in the claim. The methods and the vinyl ethers obtained are not claimed.

In a first embodiment of the process suitable for making perfluorinated vinylethers like the ones as claimed, an appropriate hydrocarbon (e.g., containing at least one 2-alkoxy propionate moiety, derivative or equivalent) precursor is provided which includes a hydro- carbon acid derivative. Such derivatives include, for example, acid fluorides, anhydrides, esters and the like. This derivative is then perfluorinated to provide a corresponding perfluorinated acid derivative intermediate. The intermediate is then converted to its corresponding perfluorinated acid metal salt and subsequently converted to the desired perfluorovinyl ether.

In a second embodiment of a process suitable for making perfluorinated vinylethers like the ones claimed, a partially fluorinated hydrocarbon precursor is provided by reacting hexafluoropropylene oxide with an alcohol or a species such as sodium alkoxide. The precursor is then fully fluorinated by replacing any remaining carbon bonded hydrogen atoms with fluorine atoms to provide a perfluorinated acid derivative intermediate. The intermediate is then converted to its corresponding per- fluorinated metal salt and subsequently converted to the desired perfluorovinyl ether.

In a third embodiment for a process suitable for making perfluorinated vinylethers like the ones claimed, a partially fluorinated hydrocarbon precursor is provided by reacting a fluorinated olefin with an alcohol, 2) fully fluorinating the precursor, 3) hydrolyzing the fluorinated precursor to an acid derivative, 4) converting the acid derivative to its corresponding perfluorinated acid metal salt, 5) converting the salt product to its corresponding perfluorovinyl ether.

In the claimed invention a perfluorovinyl ether having the formula $R_fOCF_2OCF=CF_2$ is used as comonomer wherein $R_f$ is as recited in the claim.

**Detailed Description:**

The perfluorinated vinyl ethers recited in the claim are useful in the preparation of fluoroelastomers, especially those that are used at low temperatures. Similar elastomers are known. See, for example, Uschold et al., U.S. Patent 4,513,128.

The embodiment of the process suitable for making the perfluorinated vinyl ethers like the ones as claimed selectedis not critical to the practice of the invention. However, there are certain process steps common to each of the embodiments. The process and the perflorinated vinyl ethers obtained by it are not claimed.

Fluorination of the precursors may be accomplished by either electrochemical fluorination (ECF) or direct fluorination (DF). ECF is described in U.S. Patent 2,713,593 and WO 98/50603. DF is described in U.S. Patent 5,488,142.

Conversion of the perfluorinated precursor to the metal salt is preferably accomplished by treatment with a base, e.g. saponification.

Conversion of the perfluorinated metal salt to the vinyl ether is preferably accomplished by pyrolysis. Typically this is done by drying the salt and then heating the salt to a temperature of from 170° C to 250° C with or without the presence of a solvent or other medium.

The following discussion specifically addresses three embodiments of the process suitable for making the perfluorinated vinyl ethers like the ones recited in the claim. It is not intended to limit the scope of the disclosure to these embodiments. Rather it illustrates the versatility of the process, however, the process and the vinyl ether obtained by it are not claimed.

In the first embodiment of a process suitable for making a perfluorinated vinlyether like the ones recited in the claim, the perfluorovinyl ether may be prepared by

(a) providing a hydrocarbon ester, anhydride, acid halide or acid precursor which comprises at least one 2-alkoxypropionate moiety, derivative or equivalent,
(b) fluorinating the precursor to provide a perfluorinated acid derivative intermediate,
(c) converting the perfluorinated intermediate to its perfluorinated metal salt, and
(d) pyrolyzing the perfluorinated metal salt to the corresponding perfluorovinyl ether.

More specifically, the first embodiment may be exemplified by the following synthesis sequence:

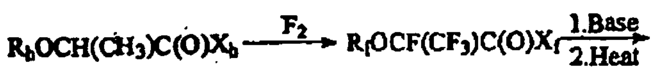


$R_fO-CF=CF_2$

where $R_h$ is a $C_1$-$C_{20}$ alkyl or aromatic group, which may be linear, branched, cyclic and which may contain additional ether linkages. The $X_h$ moiety is selected from the group consisting of $P_h$, lower alkoxy, (such as $-OCH_3$, $-OCH_2CH_3$), $OC(O)CH(CH_3)OR_h$, -F and -Cl. $R_f$ is a perauorinated version of $R_h$ and $X_f$ is a perfluorinated version of $X_h$.

In the case where a divinyl ether is desired, a precursor may be represented as

$R'_h(OCH(CH_3)C(O)X_h)_2$

where $R'_h$ is described in a similar manner to $R_h$ above except it is divalent. The $R_h$ moieties in this particular precursor may be the same or different. Such a divinyl ether is $CF_2=CFOR'_fOCF=CF_2$ wherein $R'_f$ is a perfluorinated version of $R'_h$.

In an additional possibility, the pendant methyl group of the $-CH(CH_3)COO-$ moiety may contain a chlorine atom. This chlorine atom will survive the fluorination step and may provide some reactive advantage in subsequent steps.

When $R_1$ is linear, this route opens new possibilities for the synthesis of long chain, non-branched vinyl ether in an efficient and economical way and is not limited to certain sequences in the side chain. In contrast see, for example, Masahiro et al, Daikin EP 290,848 where the molecule is limited to a repetition of the sequence $(-OCF_2CF_2CF_2-)$.

An alternate approach to the hydrocarbon precursor for this embodiment involves esters of 2-alkoxy-1-propanol. This precursor has the configuration of the ester moiety reversed when compared to the first reaction sequence listed, but is functionally equivalent to a 2-alkoxypropionate in the process of this invention. The precursor is then perfluorinated as described above. The resulting species then is dissociated to produce the perfluoro-2-alkoxy propionyl fluoride. From this point the conversion to the vinyl ether proceeds as in the first reaction sequence by converting the propionyl fluoride to the corresponding perfluorinated metal salt and pyrolyzing the salt to the corresponding perfluorovinyl ether.

An additional precursor which may be used in this route includes

$R^3OCH(CH_2Cl)CH_2OA_c$

where $R^3$ is an alkyl group which may contain additional ether linkages and

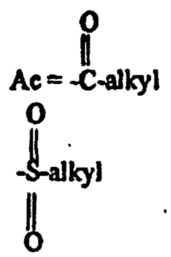

or

with the alkyl being a $C_1$-$C_6$ alkyl group. Preferably, the alkyl is a $C_1$ alkyl group that may be fluorinated. The chlorine on the pendent methyl group will also survive the fluorination process.

The hydrocarbon containing precursors are either commercially available or are easily prepared by common organic synthesis. Corresponding long chain polyether alcohols may be coupled with 2-chloropropionic acid to achieve the desired precursors, but any other method is also useful.

Examples of ester precursors for fluorination include the following precursors and reference (*) precursors:

$CH_3OCH(CH_3)COOCH_3$ (*)

$[CH_3OCH(CH_3)CO]_2O$ (*)

$CH_3OCH(CH_3)CH_2OCOCH_3$ (*)

$CH_3OCH(CH_3)CH_2OCOCF_3$ (*)

$CH_3OCH(CH_3)CH_2OSO_2CF_3$ (*)

$CH_3OCF(CF_3)COOCH_3$ (*)

$[CH_3OCF(CF_3)CO]_2O$ (*)

$CHF_2OCH(CH_3)COOCH_3$ (*)

$CH_3OCH(CH_2Cl)COOCH_3$ (*)

$HCF_2CF_2OCH(CH_3)COOCH_3$ (*)

$CF_3CH_2OCH(CH_3)COOCH_3$ (*)

$CH_3OCH_2OCH(CH_3)COOCH_3$

$C_2H_5OCH_2OCH(CH_2Cl_2)CH_2OCOCH_3$

$CH_3OCH_2CH_2OCH(CH_3)COOCH_3$ (*)

$CH_3OCH_2CH_2$ $CH_2OCH(CH_3)COOCH_3$ (*)

$CH_3OCH(CH_3)CH_2CH_2OCH(CH_3)COOCH_3$ (*)

$C_2H_5OCH_2CH_2CH_2CH_2OCH(CH_3)COOCH_3$ (*)

$CF_3OCF_2CHFCF_2OCH(CH_3)COOCH_3$ (*)

$CF_3OCHFCF_2OCH(CH_3)COOCH_3$ (*)

$CF_3CHFCF_2OCH(CH_3)COOCH_3$ (*)

$C_2H_5OC_2H_4OC_2H_4OCH(CH_3)COOCH_3$ (*)

$nC_3F_7CH_2OCF(CF_3)COCl$ (*)

$nC_3F_7CH_2OCF(CF_3)COF$ (*)

$(CH_3)_2CHCH_2OCH(CH_3)COOCH_3$ (*)

$C_8H_{17}OCH(CH_3)COOCH_3$ (*)

$C_8F_{17}C_2H_4OCH(CH_3)COOCH_3$ (*)

$C_6H_5OCH(CH_3)COOCH_3$ (*)

4-$CH_3OC_6H_4OCH(CH_3)COOCH_3$ (*)

$CF_3OCFHCF_2OCH_2OCH(CH_3)CO_2CH_3$

Fluorination of the precursors can be done by electrochemical fluorination (ECF) as previously described. ECF may be operationally simpler because only one reactor is used. However, ECF tends to give lower yields of some species because of C-O and C-C bond cleavage reactions.

In contrast, direct fluorination (OF) conducted according to U.S. Patent Nos. 5,476,974 or 5,488, 142 leads to little or no rearrangement and only minor losses attributable to cleavage reactions. Thus, in spite of the need for both a fluorine generator and a direct fluorination reactor, OF is preferred in most cases because of a higher yield of the desired product.

Some partially fluorinated precursors, such as 2,3,3,3-tetrafluoropropionic derivatives of the second embodiment give little rearrangement and less cleavage in ECF than the unfluorinated precursors.

The resulting perfluorinated esters, anhydrides or acid fluoride end groups may be converted to the corresponding hydrocarbon methyl esters with methanol if a separation step (e.g. distillation) is desired to purify the reaction product. This is typically done to separate the perfluorinated compounds from the reaction media. Either the separated product or the perfluorinated products in the reaction mixture are then saponified with bases, i.e. converted to a salt, (e.g. KOH, $Na_2CO_3$, NaOH) to give the resulting salts. The salts are then dried and pyrolyzed at temperatures from 170°C to 250°C with or without solvent to give the corresponding perfluorovinyl ether. The resulting perfluorovinyl ether is preferably purified by distillation to obtain the desired purity.

In a second embodiment of the process suitable for use in the invention a partially fluorinated hydrocarbon precursor is used. This precursor is prepared by reacting hexafluoropropylene oxide with an alcohol (such as is described

in D. Sianesi et al., J. Org. Chem., Vol. 3 (1966), p. 2312). This process comprises the steps of:

(a) providing a partially fluorinated hydrocarbon precursor by reacting hexafluoropropylene oxide with an alcohol,
(b) perfluorinating the precursor, for example, by electrochemical or direct fluorination, to provide a perfluorinated ester or acid intermediate,
(c) converting the perfluorinated intermediate to its corresponding metal salt, and
(d) pyrolyzing the perfluorinated salt at a temperature sufficient to provide a perfluorovinyl ether.

This process is represented by the following synthesis sequences.

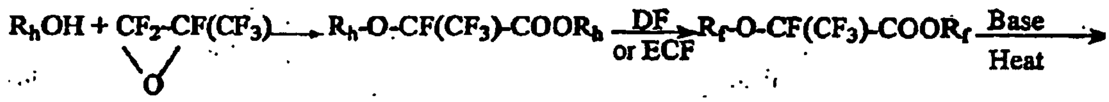


$R_fO\text{-}CF{=}CF_2$ , wherein $R_f$ and $R_h$ are as described above.

The fluorination, conversion to a salt and pyrolysis to the perfluorovinyl ether are done in a manner similar to that described in the first embodiment.

In a third embodiment of the process suitable for making perfluorinated vinyl ethers like the ones recited in the claim, a perfluorinated vinyl ether may be prepared by a process comprising the steps of:

(a) providing a partially fluorinated precursor which is a reaction product of a fluorinated olefin and an ester of lactic acid,
(b) perfluorinating the precursor, to provide a corresponding perfluorinated ester or acid derivative intermediate,
(c) converting the perfluorinated intermediate to its corresponding metal salt, and
(d) pyrolyzing the perfluorinated salt to the corresponding perfluorovinyl ether.

More specifically, the third embodiment of the process is exemplified by the following synthesis sequences.

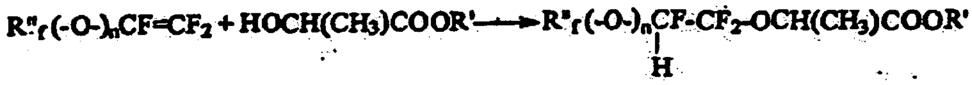

where n = 0 or 1, and R"r is F or $R_f$ when n is 0, and $R''_f$ is $R_f$ when n is 1, and R' is an C1 to C6 alkyl.

Preferred olefins for the addition to the hydrocarbon ester are tetrafluoroethylene, hexafluoropropene, perfluoro(alkyl vinyl) ethers, such as perfluoro(methyl vinyl) ether, perfluoro(propyl vinyl) ether or other perfluoro vinyl ethers as described herein. The preferred bases to catalyze the addition are alkali hydroxides KOH, NaOH, or NaOMe. Solvents for the reaction include N,N-dialkyl carboxylic acid amides, such as those disclosed in U.S. Patent No. 4,433,180 (von Wemer). The 2-alkoxy propionate moiety is supplied by the hydrocarbon ester in this embodiment.

The resulting partially fluorinated precursors are fluorinated to provide a perfluorinated acid derivative intermediate. This step may be accomplished by either ECF or DF as discussed above. The solvents for the direct fiuorination embodiments are perfluorinated compounds and/or fluorochloro compounds, e.g. perfluoromethylmorpholine, Freon-113, etc.

The perfluorinated intermediate is then converted to a metal salt. The salt is then pyrolyzed to the corresponding perfluorovinyl ether. These steps are accomplished in a manner similar to that described in the first embodiment.

Examples of perfluorinated vinyl ethers made according to one or more of the methods described above include the following compounds and reference (*) compounds

$CF_3O(CF_2)_3OCF{=}CF_2$ (*)

and

$R_f\text{-}OCF_2OCF{=}CF_2$,

including

$CF_3OCF_2OCF{=}CF_2$

and

$C_2F_5OCF_2OCF=CF_2$

**Examples:**

Reference Example 1 - Perfluoro (methoxyethyl) vinyl ether (not claimed)

Methyl 2-(methoxyethoxy) propionate was prepared by the alkylation of methoxyethanol (1500g, 19.7m) with 2-chloropropionic acid (1050g, 9.7m), using NaOH (790g, 19.?m) and tetrabutylammonium bromide (40g, 0.12m) in 1.2 liters of THF. After removal of the excess starting alcohol, methanol (965g, 30m) and HCl (382g, 10.5m) were added. Distillation gave the desired ester (832g, 5.1m, 53% yield) b.p: 100-120°C/15mm (2.0 kPa) identified by H-nmr. The ester was fluorinated in perfluoro-N-methylmorpholine (PMM) as described in U.S. Patent 5,488,142. After fluorination, methanol (850g, 26.6m) was added and distillation gave methyl perfluoro 2-(methoxyethoxy) propionate (1334g, 3.7m, 56% yield) b. p. rn-115°C, identified by 19F NMR. The fluorinated ester (500g,1.39m) was reacted with KOH (93g,1.4m) dissolved in 400g of methanol to give the corresponding salt. The salt was stripped of methanol and water at 90°C/15mm (2.0 kPa) after the addition of 21 g $K_2CO_3$ and 1000g of Fluorinert™ FC71 (from 3M Co.). Vacuum was broken and the salt decarboxylated at 180-220°C. Perfluoro (methoxyethyl) vinyl ether was distilled (299g, 1.06m, 76% yield) b. p. 46-48°C, identified by 19F NMR.

Table 1 lists perfluorovinyl ethers prepared in a manner similar to Reference Example 1 using the listed starting material. The boiling points (bp) listed were measured at atmospheric pressure.

**TABLE 1**

| Ref.Ex. | Starting Material | Product | Bp |
|---|---|---|---|
| 1 | 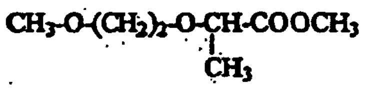 | $CF_3$-O-$(CF_2)_2$-O-CF=$CF_2$ | 48°C |
| 2 | 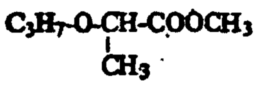 | $C_3F_7$-O-CF=$CF_2$ | 39°C |
| 3 | 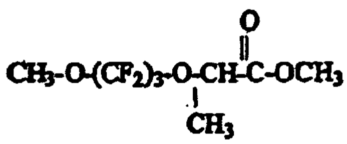 | $CF_3$-O-$(CF_2)_3$-O-CF=$CF_2$ | 64°C |

Reference Example 4 - Perfluoro(methyl vinyl) ether (not claimed)

Hexafluoropropylene oxide was reacted with methanol at room temperature to give in almost quantitative yield $CH_3$-O-CF($CF_3$)-$COOCH_3$ This product was then fluorinated in perfluoro(methyl morpholine) in a tubular reactor as described in U. S. Patent 5,488,142. The resulting perfluorinated compound was isolated and saponified with potassium hydroxide. The resulting salt was dried and pyrolyzed at 250°C to give perfluoromethylvinyl ether in a 60% molar yield.

Table 2 lists vinyl ethers produced in a manner similar to Reference Example 4, but using the listed starting alcohol. Boiling points were measured at atmospheric pressure.

TABLE2

| Ref. Ex. | Starting Alcohol | Product | Bp |
|---|---|---|---|
| 4 | $CH_3OH$ | $CF_3$-O-CF=$CF_2$ | -23°C |
| 5 | $C_3H_7OH$ | $C_3F_7$-O-CF=$CF_2$ | 36°C |
| 6 | $CH_3OCH_2CH_2OH$ | $CF_3$-O-$CF_2$-$CF_2$-O-CF=$CF_2$ | 48°C |

Reference Example 7. Perfluoro octyl vinyl ether (not claimed)

In a procedure similar to Reference Example 1, 1-octanol was converted to methyl 2-octyloxypropionate, bp 100-110C/10mm (1.3 kPa), and 764.5 g (3.54 mol) of this was fluorinated in a 20 liter reactor according to the procedure of U.S. Patent 5,476,974 in $CF_2ClCFCl_2$ solvent. Methanolysis and distillation gave 1143g (54%) $C_8$ $F_{17}OCF(CF_3)COOMe$, and saponification and pyrolysis gave $C_8F_{17}OCF=CF_2$, bp 136-137°C.

Reference Example 8. Perfluoro 3-methoxypropyl vinyl ether, $CF_3O(CF_2)_3OCF=CF_2$ (not claimed)

In a procedure similar to Reference Example 1, $CH_3O(CF_2)_3OH$ was converted to $CF_3O(CF_2)_3OCF(CF_3)COOMe$ bp 123-126°C and this to $CF_3O(CF_2)_3OCF=CF_2$ bp 62-64°C.

Reference Example 9. Perfluoro 3-methoxybutyl vinyl ether, $CF_3OCF(CF_3)C_2F_4OCF=CF_2$ (not claimed)

In a procedure similar to Reference Example 1, $CH_3OCH(CH_3)C_2$ $H_4OH$ was converted to $CF_3OCF(CF_3)C_2F_4OCF(CF_3)COOMe$ bp 145-148°C and this to $CF_3OCF(CF_3)C_2F_4OCF=CF_2$, bp 84-86°C.

Reference Example 10. Perfluoro 2,6-dimethylcyclohexyl vinyl ether (not claimed)

In a procedure similar to Reference Example 1, 2,6-dimeihylphenol was converted to 2,6-$(CF_3)_2$-cyclic-$C_6F_9OCF(CF_3)COOMe$, bp 75-80°C/3mm (0.4 kPa), and this was then converted to 2,6-$(CF_3)_2$-cyclic-$C_6F_9OCF=CF_2$ bp136-138° C.

Reference Example 11. Perfluoro ethoxyethoxyethyl vinyl ether $CF_3CF_2OC_2F_4OC_2F_4OCF=CF_2$ (not claimed)

In a procedure similar to Reference Example 1, ethoxyethoxyethanol was converted to $CF_3CF_2OC_2F_4OC_2F_4OCF(CF_3)COOMe$, bp 170-175°C, and this to $CF_3CF_2OC_2$ $F_4OC_2$ $F_4OCF=CF_2$, bp 92-95°C.

Reference Example 12. Perfluoro propyl vinyl ether, $C_3F_7OCF=CF_2$ (not claimed)

In a procedure similar to Reference Example 1, n-propanol was converted to 2-propoxypropionic acid. This upon treatment with acetyl chloride afforded the anhydride $(C_3H_7OCH(CH_3)CO)_2O$, bp 118-22°C/15 mm (2.0 kPa), which upon direct fluorination according to U.S. Patent 5,488,142 gave $(C_3F_7OCF(CF_3)\ CO)_2O$, bp 186-190°C. The perfluoroanhydride was methanolysed to $C_3F_7OCF(CF_3)COOMe$, bp 118-122°C and converted to $C_3F_7OCF=CF_2$, bp 36°C.

Reference Example 13. Perfluoro methyl vinyl ether, $CF_3OCF=CF_2$ (not claimed)

In a procedure similar to Reference Example 12, 2-methoxypropionic acid was converted to the anhydride $(CF_3OCF(CF_3)CO)_2O$. This was methanolysed to $CF_3OCF(CF_3)COOMe$ bp 58-62°C and this converted to $CF_3OCF=CF_2$ bp -23°C.

Reference Example 14. Perfluoro methyl vinyl ether from 2-methoxy-1-propyl acetate (not claimed)

NaH (60% in mineral oil, 8.0g, 0.2 mol) was washed with hexane, slurried in THF, and treated dropwise with 26.4g (0.2 mol) 1-t-butoxy-2-propanol (Aldrich Chemical) with ice cooling. The mixture was stirred at reflux one hr, chilled in ice, and treated with 19 ml (0.2 mol) dimethyl sulfate. After heating at reflux for 3 hr, the mixture was filtered, concentrated, and distilled to a head temperature of 85°C. The residue (23.9g yellow liquid) was >90% pure 1-t-butoxy-2-methoxypropane by 1H NMR analysis. Of this, 10.0g was mixed with 0.05g fused zinc chloride and 15 ml dichloromethane, chilled in ice, and treated slowly with 6.0g acetyl chloride. On warming to room temperature, gas evolution was noted. The mixture was distilled to 8.0g at about 50°C/14 Torr. 1H NMR and gc/ms confirmed 2-methoxy-1-propyl acetate in >90% purity, the main impurity being the product derived from the minor regioisomer 2-t-butoxy-1-propanol (present at about 7% in the starting material). This ester was direct fluorinated as described in U.S. Patent 5,488, 142. The resulting solution was analyzed by $^{19}F$ NMR to contain $CF_3OCF(CF_3)CF_2OCOCF_3$ in 43% yield. Methanalysis gave the same methyl ester as Reference Example 13. One could then convert to the perfluorovinyl ether as described above.

Reference Example 15. Perfluoro methyl vinyl ether from methyl 2-methoxypropionate (not claimed)

Methyl 2-methoxypropionate (520.3g) was fluorinated in an ECF cell as described in U.S. Patent 2,713,593

equipped with -40°C and -80°C condensers. The yield of $CF_3OCF(CF_3)COF$ was estimated as 17%. One could then convert this to perfluoromethylvinyl ether as described above.

Example 1. Perfluoro ethoxymethyl vinyl ether, $C_2F_5OCF_2OCF{=}CF_2$ (not claimed)

Ethyl 2-ethoxymethoxypropionate was made by stirring and heating a mixture of 1007g (8.5 mol) ethyl lactate, 2500 ml (20.2 mol) diethoxymethane (DEM), and 5.0g toluenesulfonic acid hydrate under nitrogen while distilling off a mixture of ethanol and DEM. Fresh DEM was periodically added. After 12 hr, the mixture was cooled, washed with very dilute NaOH, and the product distilled to 1232g (7.5 mol, 88%) bp 90°C/20 mm (2.7 kPa). This ester was direct fluorinated as described in U.S. Patent 5,488, 142 and the product treated with methanol to give $C_2F_5OCF_2OCF(CF_3)$ COOMe bp 107-112°C in 30% yield. A crude sample (2342g, 57% assay, remainder PMM and $CF_3COOMe$) was added to a stirred solution of 300g NaOH in 800g water at about 30°C. After stirring 18 hr, the mixture was treated with 1 liter 50% sulfuric acid and the lower phase distilled to 718g bp 60°C/l mm (0.13 kPa) as the carboxylic acid. A second batch of 2406g crude ester yielded 896g, and rewashing of the combined distillation residues with 50% sulfuric acid yield an additional 412g, for a total yield of 2029g of the carboxylic acid. Of this 1000g was mixed with 500g powdered sodium carbonate in 2.5 l. acetone for 24 hr, filtered and stripped to give $C_2F_5OCF_2OCF(CF_3)COONa$, a tan solid. A solution of 365.8g in 200 ml diethyl ether was mixed with 250 ml Fluorinert FC-71 (3M Co.), stripped at 20 mm (2.7 kPa) for 20 min, and heated to 224°C, at which time volatile product began collecting in the dry-ice cooled traps. Heating was continued for 3 hr, final temperature was 245°C. The product, the corresponding perfluorovinyl ether with a bp of 46°C, was recovered by fractionation.

Example 2. Perfluoro ethoxymethyl vinyl ether from 3-chloro-2-(ethoxymethoxy)-1-propyl acetate (not claimed)

A mixture of 10.0g $FeCl_3$ and 276 ml acetic acid was treated slowly with 312 ml epichlorohydrin at 5 to 15°C (dry-ice bath). The product was mixed with 5.1g NaOAc and filtered to 497.1 g amber liquid, a 87-13 mixture of $HOCH(CH_2Cl)CH_2OAc$ and $AcOCH(CH_2Cl)CH_2OH$. A solution of 304g of the above mixture and 200g $ClCH_2OEt$ in 600 ml methylene chloride was chilled in ice while adding 260 N,N-diisopropyl ethylamine. Distillation yielded 257.0g bp 80°C/1.2mm (0.17 kPa). The major components were identified by gc/ms, $^1H$-NMR, and $^{13}C$-NMR as $C_2H_5OCH_2OCH(CH_2Cl)CH_2OAc$ (65%), $AcOCH(CH_2Cl)CH_2OCH_2OC_2H_5$ (18%) and starting alcohols (13%). Fluorination according to U.S. Patent 5,488,142 and distillation of the PMM solvent left 184.1g perfluorinated ester as residue. This was treated with about 0.5 ml pyridine, according to U.S. Patent 5,46-6,877, with vigorous outgassing and formation of $C_2F_5OCF_2OCF(CF_2Cl)COF$, distilled to 90.2g bp 85-95°C. $^{19}F$-NMR shows this to be 73% pure. One could then convert this to the perfluorovinyl ether as described for other 3-chloro-perfluoro-(2-alkoxypropionates) in U.S. Patent 5,449,825.

Reference Example 16. Perfluoro methyl vinyl ether (not claimed)

Hexafluoropropylene oxide (HFPO, 300g) was added to 50g of 25% sodium methoxide in methanol plus 450g methanol at room temperature in a modification of the procedure of (J. Org. Chem. 31, 2312 (1960) to give $CH_3OCF(CF_3)COOMe$ bp 110-118°C. This was direct fluorinated according to U.S. Patent 5,488, 142 and the product was methanolysed to give the same ester as in Example Reference 11. One could then convert this to the perfluorovinyl ether as described above.

Reference Example 17. Perfluoro methyl vinyl ether (not claimed)

$CH_3OCF(CF_3)COOMe$ from Example 15 was fluorinated in an ECF cell according to U.S. Patent 2,713,593 to give in 21% yield $CF_3OCF(CF_3)COF$. One could then convert this to the perfluorovinyl ether as described above.

Reference Example 18. Perfluoro methyl vinyl ether via 2-methoxy-2,3,3,3-tetrafluoropropionic acid anhydride (not claimed)

The adduct of HFPO and methanol from Reference Example 15 was hydrolysed to the carboxylic acid and dehydrated with $P_2O_5$ to give 2-methoxy-2,3,3,3-tetrafluoropropionic acid anhydride, bp 144-146° C. This was direct fluorinated according to U.S. Patent 5,488,142 to give the perfluoro anhydride bp 85-88° C, methanolysis of which yielded $CF_3OCF(CF_3)COOMe$ bp 76-78° C which was converted to $CF_3\text{-}O\text{-}CF{=}CF_2$ bp -23° C.

Reference Example 19. Perfluoro propyl vinyl ether (not claimed)

Hexafluoropropylene (36g) was added to 20.8g methyl lactate and about 0.2g KF in 50 ml DMF at 20-30°C.

Distillation yielded a mixture of esters bp 64-76°C/ 55mm (7.3 kPa). This was fluorinated in an ECF cell according to U.S. Patent 2,713,593 to give in about 25% yield a 2:1 mixture of $C_3F_7OCF(CF_3)COF$ and $C_3F_7OC_2\ F_4COF$. One could then convert these to the corresponding perfluorovinyl ethers as described above.

## Claims

1. A fluoroelastomer obtainable by using a perfluorovinylether comonomer having the formula $R_fOCF_2OCF=CF_2$ wherein $R_f$ is $-CF_2CF_3$ or $-CF_3$.

## Patentansprüche

1. Fluorpolymer, das durch Verwendung eines Perfluorvinylether-Comonomers der Formel $R_fOCF_2OCF=CF_2$, worin $R_f$ für - $CF_2CF_3$ oder $-CF_3$ steht, erhältlich ist.

## Revendications

1. Fluoroélastomère pouvant être obtenu en utilisant un comonomère d'éther perfluorovinylique de formule $R_fOCF_2OCF=CF_2$, dans lequel $R_f$ est $-CF_2CF_3$ ou $-CF_3$.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4273728 A, Krespan, Carl G., DuPont de Nemours
- US 5326917 A, DuPont de Nemours
- US 4513128 A, Uschold
- US 5350497 A, Hung
- US 2713593 A
- WO 9850603 A
- US 5488142 A
- EP 290848 A, Daikin
- US 5476974 A
- US 4433180 A
- US 5466877 A
- US 5449825 A


### Non-patent literature cited in the description


- Modem Fluoropolymers. **JOHN SCHEIRS.** Polymer Science. Wiley Series, 1997
- **EMEL 'YANOV et al.** *Zh. Org. Khim,* 1994, vol. 30 (8), 1266-70
- **J. SCHEIRS.** Modem Fluoropolymers. Wiley, 1997, 376-378
- **D. SIANESI et al.** *J. Org. Chem.,* 1966, vol. 3, 2312
- *J. Org. Chem.,* 1960, vol. 31, 2312